# EUROPEAN PATENT SPECIFICATION

(11) **EP 2 140 908 B1**
(45) Date of publication and mention of the grant of the patent: **19.10.2016**
(21) Application number: 09163838.7
(22) Date of filing: 26.06.2009
(51) Int. Cl.: A61N 1/36, H04R 25/00, A61N 1/375

(54) **Devices for hearing impaired persons**
Vorrichtungen für hörgeschädigte Menschen
Dispositifs pour personnes dont l'audition est altérée

(30) Priority: 02.07.2008 AU 2008903416
(43) Date of publication of application: 06.01.2010
(73) Proprietor: Cochlear Limited, Macquarie University, NSW 2109 (AU)
(72) Inventor: Meskens, Werner, 1745 Opwijk (BE)
(74) Representative: Grünecker Patent- und Rechtsanwälte PartG mbB

(56) References cited:
- WO-A2-98/26629
- US-A1- 2002 012 438
- US-A1- 2002 071 581
- US-B1- 6 231 604

## Description

### Field of the Invention

The present description relates to a medical device and in particular to a medical device that relies on use of transcutaneous wireless links between external and implantable components, such as auditory prostheses. The present invention is set out in the appended claims.

### Background of the Invention

Depending on the degree of hearing loss in a range from mild to acquired or congenital deafness, and depending on the cause for such hearing losses, which can lie in the region of the ear which conducts the sound wave (ear drum, middle ear), in the inner ear (cochlea), or in the auditory nerve or central auditory processing, a range of auditory prostheses are available in order to improve hearing.

Basically there are two different hearing aid principles: acoustic mechanical stimulation, or electrical stimulation. With acoustic mechanical stimulation, sound is amplified in various ways and delivered to the inner ear through the column of air to the ear drum, or direct delivery to the ossicles of the middle ear as mechanical energy. Acoustic mechanical stimulation requires the cochlea, hair cells, and auditory nerve provide sufficient functionality. When the functionality of the cochlea, and in particular, the hair cells, deteriorates the efficiency of the acoustic mechanical stimulation of the hair cells decreases.

At a point where acoustic mechanical stimulation is no longer effective at all, the auditory nerve can be stimulated directly by applying electrical signals representing the sound to the auditory nerve that extend from the cochlea. Electrical stimulation does not require that the structure of the cochlea and the hair cells be intact. It is only necessary that the auditory nerve, as well as the central processing centres, are sufficiently intact. With electrical stimulation, the stimulating electrodes should be placed as close as possible to the nerve endings of the auditory nerve. This occurs optimally when an electrode carrier is inserted into the cochlea. Such stimulating electrodes are part of an auditory prosthesis that is commonly known as a cochlear implant. Cochlear implants can comprise an external component, such as a speech processor unit, and an implantable component, such as a stimulator unit. Such an exemplary cochlear implant is shown, for example, in U.S. Patent Publication No. 2002/0012438 to Leysieffer et al., which is directed to a system for rehabilitation of a hearing disorder that includes an implanted hearing system that delivers mechanical or electrical stimulation to a recipient. More specifically, Leysieffer discloses a system that includes an implant attached to one or both ears of a recipient where the implanted hearing system may communicate wirelessly with a remote control, which may be used to control the implant functions of the implant(s), or with a second implanted hearing system. The external component typically comprises a casing, a microphone, a speech processor that converts detected sounds into coded signals and a power source. The implantable component receives the coded signals and power from the external component and outputs a stimulation signal to an electrode assembly which applies electrical stimulation to the auditory system of the implantee producing a hearing sensation corresponding to the original detected sound.

Communication between the external component and the implantable component can be provided by a radio frequency (RF) magnetic induction link comprising an inductively coupled external antenna coil and an internal implanted antenna coil. This RF link provides transcutaneous transmission of the coded signals to, and also typically from, the implantable component and can also serve to provide power to the implantable component. Implantable components having an onboard rechargeable battery have also been proposed. Such prostheses can utilise more than one type of external component or work together with other external or implantable components.

A general problem of such cochlear implants is that this procedure may cause the destruction of structures of the inner ear which may still be functioning. Therefore, this technique is not used when there is significant residual hearing.

Since the choice between acoustic mechanical stimulation and electrical stimulation depends upon the degree of hearing loss, there is gap for the medical care of hearing impaired persons, where there is no optimal treatment and respectively suitable hearing aid devices.

For instance there can occur the situation, where there is sufficient reserve functionality of the cochlea, but there is not sufficient sound conduction in the air and liquid based conduction parts of the ear. Since inserting electrical stimulation electrodes may destroy the structures of the inner ear, and since electrical stimulation is indicated only for nearly complete deafness, a cochlear implant does not fall within the medical indication for such a measure, and there remains no appropriate treatment for the patient.

Another aspect is that a person could loose his/her ability for understanding speech due to partial damages of the hair cells in the cochlea due to aging, extreme noises. Partial damages mean that the sensitivity to particular frequencies drop out so that a useful speech understanding is no longer possible. On the other hand, electrical stimulation is particularly effective at the nerve endings at the basal region of the cochlea. The fact that electrical stimulation improves higher tones while low frequencies are not as well reached is the result of inability to insert the electrode carrier to the apex of the cochlea. Thus, with a high percentage of the moderately to severely hearing impaired, previously available acoustical hearing aids did not result in a satisfactory speech understanding due to hearing loss in the high frequencies. Electrical stimulation in the sense of an electrode inserted into the cochlea was also not an option for these patients due to the loss of residual hearing associated with this procedure.

US 6 231 604 B suggests therefore to combine both acoustic mechanical stimulation and electrical stimulation. As has been proven by earlier models of cochlear implants, extracochlear electrical stimulation can be effective, although the results achieved with complete deafness are not comparable to those achieved with intra-cochlear stimulation. When used in combination with acoustic mechanical stimulation, extracochlear electrical stimulation with the residual hearing may be sufficient.

There remain however several problems. At first, US 6 231 604 B suggests to supply the acoustic mechanical stimulation unit and the electrical stimulation unit only with frequency subranges of the audio frequency spectrum. That means that the electronics has to be configured individually to each patient because, dependent on the type of hearing loss, the frequency bands have to be adjusted. This also means that the data streams to the stimulation units become unbalanced. In addition, the device of prior art document US 6 231 604 B suggests only to combine acoustic mechanical stimulation and electrical stimulation in one ear, because the device of prior art document US 6 231 604 B considers only the particular situation, where the cochlea is electrically stimulated externally in a suitable frequency subrange and the acoustic mechanical stimulation supports the hearing aid in another suitable frequency subrange. Due to the unbalanced data streams in the device of prior art document US 6 231 604 B, a general application of this device in both ears is not possible. Moreover, prior art document US 6 231 604 B allows only a combination of acoustic mechanical stimulation and electrical stimulation. This does not consider cases, where the cochlea is fully intact and does not need electrical stimulation. For example, there are situations, where there are several problems in the air and liquid conduction paths which cannot be solved by a single stimulation approach. For instance a bone-anchored hearing aid might not be sufficient to achieve speech understanding.

In view of these problems there is an object of this invention to provide an auditory prosthesis that can more flexible address difficult hearing losses whereby the adaptation to the patient is cost effective.

### Summary of the Invention

The present invention is set out in the appended claims. The examples, aspects or embodiments of the present description that do not fall within the scope of said claims are provided for illustration purposes only and do not form part of the invention.

At least some of the problems discussed above are solved by an auditory prosthesis as set forth in claim 1. The auditory prosthesis comprises:
(I) a processor unit comprising:
   (a) a microphone for receiving and converting detected sounds into an output signal;
   (b) a processor that converts and processes the output signal into a first digital signal; and
   (c) a first transceiver or first transmitter that wirelessly transmits the first digital signal;
(II) an implantable first stimulation module adapted to output a first stimulating signal representative of the detected sound; and
(III) at least one second stimulation module adapted to output a second stimulating signal based on wirelessly received sound information according to the first digital signal based on wirelessly received sound information according to the first digital signal,
wherein at least one of the first and second stimulation modules comprises at least one of a transceiver and a receiver that receives and processes the first digital signal to achieve at least one of said first and second stimulating signal.

Thus, a very flexible auditory prosthesis can be achieved which can be easily configured to address complex hearing losses. For instance, the auditory prosthesis can be applied to only one ear or to both ears. A wireless connection between the units allows a flexible usage of one ore more second stimulation modules to support the stimulation of the first stimulation module. If e.g. the first stimulation module is a cochlear implant, an in-the-ear second stimulation module in the second ear with residual hearing ability can improve speech understanding. Wireless links can be used for energy and data transfer which also improves the usability of the hearing prosthesis because of improved wearing comfort and because no cable has to be adapted to a particular user. Since the modules may be provided with processors, data communication and configuration may be adjusted and tuned e.g. when components have to be changed or when the hearing loss is progressive. Moreover, the combination of the implanted first stimulation module with the second not implanted stimulation module compensates deficiencies of the respective mutual stimulation module. For instance, the acoustic mechanical stimulation of the second stimulation module can compensate deficiencies of the implanted first stimulation module e.g. due to imprecisely implanted cochlea implants. The combination of stimulation principles can also support learning to interpret the nerve stimuli.

The auditory prosthesis allows several basic configurations.

In one embodiment the implantable first stimulation module comprises at least a second transceiver or first receiver that wirelessly receives the first digital signal and processes the first digital signal into said first stimulating signal representative of the detected sound, and the at least one second stimulation module comprises at least a third transceiver or second receiver that wirelessly receives and processes said first digital signal into a second stimulating signal representative of the detected sound.

Thus, the auditory prosthesis has three different and intelligent parts which can be individually replaced according to the requirements, e.g. when a bigger main battery is required, or when an additional in-the-ear module is required.

According to an alternative embodiment, the implantable first stimulation module includes the processor unit, and the at least one second stimulation module comprises at least a third transceiver or second receiver that wirelessly receives and processes said first digital signal into a second stimulating signal representative of the detected sound.

In this embodiment, the processor unit and the implantable first stimulation module are combined and allows a more resource saving solution.

In a further alternative embodiment, the implantable first stimulation module comprises at least a second transceiver or first receiver that wirelessly receives the first digital signal and processes the first digital signal into said first stimulating signal and a second digital signal representative of the detected sound, and the at least one second stimulation module comprises at least a third transceiver or second receiver that wirelessly receives and processes said second digital signal into a second stimulating signal representative of the detected sound. Thus, the first stimulation module achieves the master control of the auditory prosthesis, so that the first stimulation module can appropriately distribute the stimulation signals to respective actuators.

According to another embodiment, the microphone, processor and first transceiver or first transmitter are part of an external component having a housing, the external component is selected from group comprising a behind-the-ear (BTE) unit, an in-the-ear (ITE) unit, a pinna attached unit, or a body worn unit.

According to a further embodiment, the first stimulation module comprises at least one of a cochlear implant, an actuator arrangement configured to provide direct stimulation to one or more hearing structures of an implantee, a direct acoustic cochlear actuator (DACS) unit, and a bone anchored hearing aid (BAHA).

According to an just another embodiment, the cochlear implant includes an intracochlear electrode assembly comprising a carrier member having a leading end that is insertable into a cochlea of the implantee and a trailing end, the carrier being formed of an elastomeric material and having a plurality of biocompatible electrodes mounted thereon.

According to an another embodiment, the actuator arrangement comprises at least one extracochlear actuator that is arrangable to provide at least one of mechanical excitation to fluid-filled inner-ear spaces, and electrical excitation of the auditory nerve at the outside of the cochlea. This embodiment enables a stimulation of the cochlea without destroying the intact hair cell structures. The combination with the second stimulation unit compensates the impreciseness of the external stimulation of the cochlea.

According to a further embodiment, the bone-anchored hearing aid comprises a bone transmitting vibrator that is connected directly to the bone using a skin-penetrating and bone-anchored implant of a biocompatible material.

According to an embodiment, the second stimulation module comprises an acoustic/mechanical stimulation module that delivers mechanical stimulation in a range of audio frequencies, wherein the acoustic/mechanical stimulation module is selected from the group comprising: a completely-in-canal (CIC) unit that is shaped to fit into the ear canal of the implantee; an in-the-ear (ITE) unit that fits within the ear bowl; an in-the-canal (ITC) unit; and a micro-canal (MIC) unit.

According to quite another embodiment, each of the respective transceivers, transmitters or receivers are provided with one or more antennae to provide wireless links between the components of the prosthesis, wherein at least one of the wireless links is provided preferably by a time domain multiple access (TDMA) system.

According to yet another embodiment, a wireless link from the external component is used to power at least one of the first and second stimulation module and/or recharge a power source that is on-board the first or second stimulation module.

According to a further embodiment, another one of the one or more antennae provides a transcutaneous wireless link with a separate antenna on the implantable first stimulation module to allow transmission and reception of said first and second digital signals.

According to an embodiment thereof, said separate antenna of the first stimulation module is used to transcutaneously re-transmit the first and second digital signal to the second stimulation module.

### Brief Description of the Drawings

By way of example only, embodiments are now described with reference to the accompanying drawings, in which:
Fig. 1 depicts one embodiment of an auditory prosthesis according to the present description.
Fig. 2 depicts an embodiment of the second stimulation module used in the prosthesis of Fig. 1;
Fig. 3 depicts another embodiment of an auditory prosthesis according to the present description.
Fig. 4 depicts a still further embodiment of an auditory prosthesis according to the present description, and
Fig. 5 depicts yet a different embodiment of an auditory prosthesis according to the present description.

### Preferred Mode of Carrying out the Invention

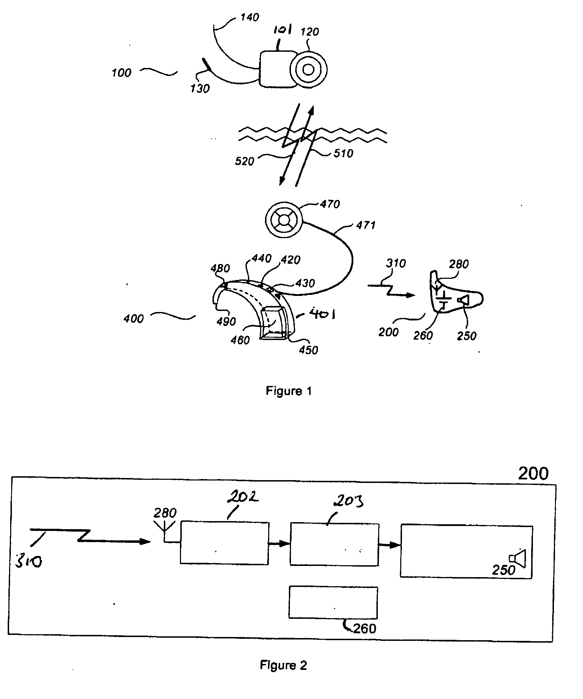

One embodiment of an auditory prosthesis is depicted in Fig. 1. The prosthesis comprises an external component 400 in the form of a behind-the ear (BTE) unit. The unit 400 has a main housing 401, a carrier for plug-in modules such as power sources (e.g. battery packs 460), and an ear hook 490. The battery pack 460 can comprise Zn-Air batteries or a rechargeable Lithium-ion battery pack. The housing 401 can be made of a suitable material, such as metallic material, a ceramic material, a polymeric material, or a combination of materials.

Mounted on the housing 401 is a microphone 440 for receiving and converting detected sounds into an output signal. The housing 401 also has a human interface in the form of control buttons 420, a screen display 430, and digital and analogue inputs and outputs 450.

The housing 401 also contains a processor that converts and processes the output signal from the microphone 440 into a digital signal and a first transceiver or first transmitter. In conjunction with the antenna coil or closed wire loop, that is part of headpiece 470 connected to the housing 401 by cable 471, the first transceiver or first transmitter wirelessly transmits the digital signal to the implantable first stimulation module 100.

While the external component 400 is depicted as a BTE unit, it will be appreciated that the component 400 could be resized in other shapes such as an in-the-ear (ITE) unit, a pinna attached unit, or a body worn unit.

The depicted first stimulation module 100 in Fig. 1 is the implantable component of a cochlear implant. The implantable component of the cochlear implant can comprise a housing 101 for a second transceiver or first receiver and a stimulator unit. The implant 100 further comprises at least one antenna 120, depicted in the form of an antenna coil, and an intracochlear electrode assembly 140 that delivers stimulation to the auditory nerve of the implantee so producing a hearing sensation corresponding or representative of the original detected sound.

The intracochlear electrode assembly 140 can comprise a carrier member having a leading end that is insertable into a cochlea of the implantee and a trailing end. The carrier can be formed of an elastomeric material, for example a silicone rubber material. The carrier member can have a plurality of electrodes mounted thereon. In one embodiment, the electrodes are mounted in a longitudinal array. Each of the electrodes can have at least one wire, for example two, extending from each electrode back towards the trailing end of the carrier member and then through a cable that extends back to the housing of the implantable component.

The carrier can have 22 electrodes. In another embodiment, the carrier can have 30 electrodes. Other numbers of electrodes can be utilised, including less than 20 electrodes, between 20 and 30 electrodes, and more 30 electrodes. The electrodes can be formed from a biocompatible electrically conducting material, such as platinum.

As depicted, the implantable component 100 can optionally have a second electrode assembly 130 extending from the housing. The second electrode assembly 130 may have one or more electrodes. This electrode assembly can be mounted within or external the cochlea of the implantee.

In this embodiment, the housing 101 of the implantable component 100 can be positioned subcutaneously within a recess in the temporal bone of the implantee. The housing 101 can be formed from a biocompatible material, for example, titanium. This housing 101 can in turn be encapsulated in an electrically insulating surround. In a further embodiment, one or more hermetic and electrically insulated feedthroughs can be provided in the housing 101. The feedthroughs provide signal connection through the housing to the at least one antenna coil 120 and/or the first and/or second electrode assembly 130,140. Power for the implantable component 100 can be provided using the transcutaneous magnetic induction radio frequency link 510. While not depicted in Fig. 1, the implantable component 100 can have an on-board rechargeable power source that is usable for example when the external component 400 is not being used. The forward and backward telemetry links 510,520 between the BTE unit 400 and the implantable component 100 are based on magnetic coupling, utilising the non-propagating quasi-static magnetic field. The magnetic field has a relatively high roll-off behaviour as a function of distance.

As depicted, a second digital wireless link 310 is provided between the BTE unit 400 and a second stimulation module 200. This wireless link can be based on a modulated electromagnetic field, quasi-static magnetic field, magnetic induction radio, ultrasound, electrostatic field or use of optical transmission, for example infrared. In this embodiment, an antenna 480 is provided on the ear hook 490. It will be appreciated that the antenna 480 could be on other locations in the unit 400.

The second stimulation module has a third transceiver or second receiver 202 that receives and processes the transmitted digital signal into a second stimulating signal representative of the detected sound.

The second stimulation module 200 can comprise an acoustic/mechanical stimulation module that delivers mechanical stimulation in a range of audio frequencies. In the embodiment depicted in Fig. 1, the acoustic/mechanical stimulation module 200 comprises an in-the-ear (ITE) unit that fits within the ear bowl. In one embodiment, the ITE unit 200 can partially or wholly seal the ear bowl. While not depicted in Fig. 1, the module 200 could comprise a completely-in-canal (CIC) unit that is shaped to fit into the ear canal of the implantee, with the CIC unit being positionable in the ear canal so as to touch the bony portion of the ear canal. In another embodiment, the acoustic/mechanical stimulation module 200 could comprise an in-the-canal (ITC) unit, with the ITC unit being sized to not fit the ear bowl but instead be positionable just outside the ear canal. In yet another embodiment, the acoustic/mechanical stimulation module 200 could comprise a micro-canal (MIC) unit. The MIC unit is typically slightly smaller in size than the ITC unit.

In addition to the third transceiver 202, the second stimulation module 200 has an antenna 280 that feeds signals to and/or from the third transceiver 202, an on-board processor 203, a power source 260 and an electro-acoustic transducer 250. The power source 260 can comprise one or more batteries. The electro-acoustic transducer 250 can comprise a speaker. If size permits, one or more controls can be provided on the second stimulation module 200.

The respective transceivers and antennae of the hearing prosthesis can operate together to provide wireless links between the components. Some or all of the antennae can be formed so as to provide magnetic induction radio frequency links or electromagnetic radio frequency links.. In another embodiment, the wireless link can be provided by a time domain multiple access (TDMA) system.

While not depicted in Fig. 1, in another embodiment, the first stimulation module could instead comprise an actuator arrangement configured to provide direct stimulation to one or more hearing structures of the implantee representative of the original detected sound. For example, the actuator could comprise at least one extracochlear actuator and be configured to provide excitation to fluid-filled inner-ear spaces of the implantee. In another embodiment, the actuator could comprise at least one intracochlear array of actuators. The actuator can comprise one or more mechanical actuation elements. The first stimulation module could also comprise a direct acoustic cochlear actuator (DACS) unit.

In a still further embodiment that is not depicted in Fig. 1, the first stimulation module could comprise a bone anchored hearing aid (or BAHA), with the hearing aid comprising a bone transmitting vibrator that is connected directly to the bone using a skin-penetrating and bone-anchored implant of a suitable biocompatible material, for example titanium.

In one arrangement, the first and at least second stimulating signals output by the implantable module 100 and the stimulation module 200, respectively, can be provided to the same ear. In another embodiment, the first stimulating signal can be provided to one ear and the second stimulating signal to the other ear.

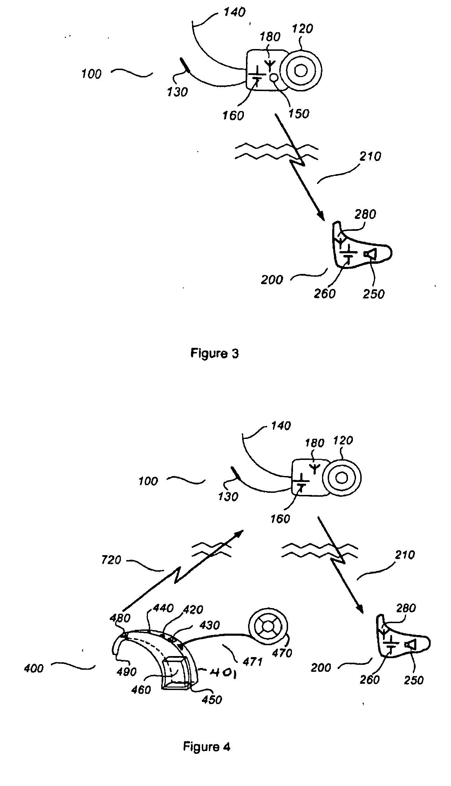

Fig. 3 depicts another embodiment of an auditory prosthesis comprising an fully implantable first stimulation module 100 (again depicted as a cochlear implant) that has on-board a power source 160, a microphone 150 for receiving and converting detected sounds into an output signal, a processor that converts the output signal into a first stimulating signal representative of the detected sound, and a first transceiver or first transmitter that works in conjunction with an antenna 180 to at least wirelessly and transcutaneously transmit the digital signal through link 210.

The prosthesis of Fig. 3 also has a second stimulation module 200 (again depicted as an ITE unit) having an antenna 280 and at least a second transceiver or first receiver that receives and processes the transmitted digital signal into a second stimulating signal representative of the detected sound.

In this embodiment, the first stimulation module 100 is fully implantable and is configured to operate, at least for a period of time, not in conjunction with any external component. The first stimulation module of Fig. 3 can have the features of the first stimulation module as defined with respect to the first aspect but modified as necessary so as to be able to operate in such a stand-alone manner.

When an external component is available and mounted appropriately, the implantable stimulation module 100 of Fig. 3 can work in conjunction with that external component. For example, if the external component has a microphone and/or processor, the output of the external processor can be used instead of the on-board microphone 150 and/or processor. When the external component comprises or has an on-board and suitable power source, the implantable component can be configured to preferentially draw power from the external component to power its operation and/or re-charge the on-board power source 160.

While a single antenna can be used, the embodiment of Fig. 3 depicts the module 100 having two antennae. One of the antennae 120 again comprises a coil antenna and can be used when necessary as part of a radio frequency magnetic induction link with a coil antenna of an external component. The other antenna 180, as already described, provides a transcutaneous wireless link 210 to the second stimulation module 200.

While depicted as an ITE unit, the second stimulation module 200 can be a CIC unit, an ITC unit, or a MIC unit. The second stimulation module 200 can also be operable to work in conjunction with the first stimulation module 100 on the same ear of the implantee or be operable to work on separate ears of the implantee. Further, while module 100 is depicted as a cochlear implant, the module 100 could be, as described above with reference to Fig. 1, another unit such as a DACS or BAHA unit.

Fig. 4 depicts yet another embodiment of an auditory prosthesis. This prosthesis comprises a BTE unit 400 again having a microphone 440 for receiving and converting detected sounds into an output signal. The housing 401 of the BTE unit 400 houses a processor that converts and processes the output signal into a digital signal and a first transceiver that at least wirelessly transmits the digital signal through additional antenna 480 via link 720 to an implantable first stimulation module 100.

The module 100 is again an implantable component of a cochlear implant and has a second transceiver or first receiver that receives the transmitted digital signal from the first transceiver or first transmitter and processes this signal into a first stimulating signal representative of the detected sound. This is delivered to the cochlea through electrode arrays 140,130.

In this embodiment, the implantable module 100 also re-transmits through antenna 180 said transmitted digital signal received from the first component 400. This re-transmitted signal 210 can be detected by the antenna 280 of the second stimulation module 200 which again has at least a transceiver that receives and processes the re-transmitted digital signal received from the first stimulation module 100 into a second stimulating signal representative of the detected sound.

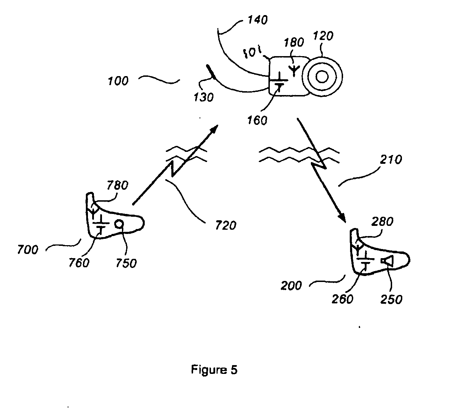

While depicted as a BTE unit in Fig. 4, it will be appreciated from Fig. 5 that the first component can comprise an in-the-ear unit 700, having a microphone 750, power source 760 and antenna 780 providing transcutaneous wireless link 720.

In Fig. 4, the BTE unit still also has coil antenna 470 thereby providing a separate radio frequency magnetic induction link with the coil antenna 120 of the first stimulation module 100. This transcutaneous link can be used to power the first stimulation module 100 and/or recharge a power source 160 that is on-board the first stimulation module 100.

In the embodiments depicted in Figs. 4 and 5, the first stimulation module 100 can have one, some or all of the features of the first stimulation module as depicted in Figs 1 and 3. It will also be appreciated that the first stimulation module 100 can comprise an implantable component of a cochlear implant, a direct acoustic cochlear actuator (DACS) unit, or a bone anchored hearing aid (or BAHA).

While depicted as an ITE unit, the second stimulation module 200 of Figs 4 and 5 can be a CIC unit, an ITC unit, or a MIC unit. The second stimulation module 200 can also be operable to work in conjunction with the first stimulation module 100 on the same ear of the implantee or be operable to work on separate ears of the implantee.

In summary according to one aspect, the present description is an auditory prosthesis comprising:
a microphone for receiving and converting detected sounds into an output signal;
a processor that converts and processes the output signal into a digital signal;
a first transceiver or first transmitter that wirelessly transmits the digital signal;
an implantable first stimulation module having at least a second transceiver or first receiver that receives and processes the transmitted digital signal into a first stimulating signal representative of the detected sound; and
at least a second stimulation module having at least a third transceiver or second receiver that receives and processes the transmitted digital signal into a second stimulating signal representative of the detected sound.

In this aspect, the microphone can be positioned on or in a housing. The housing can house the processor and/or the first transceiver or first transmitter. The housing for the processor and/or the first transceiver or first transmitter can be part of an external component worn externally by a user of the prosthesis. For example, the housing can be a behind-the-ear (BTE) unit, an in-the-ear (ITE) unit, a pinna attached unit, or a body worn unit. The BTE unit can have an ear-hook. The first transceiver or first transmitter can work in conjunction with an antenna coil that can be mounted on or in the housing or be linked by a cable back to the housing for the processor and/or the first transceiver or first transmitter. The antenna coil can form part of a magnetic induction radio frequency link with an antenna of the first stimulation module. The first transceiver or first transmitter can also work in conjunction with an additional antenna that is mounted on or in the housing. The additional antenna can transmit to and receive digital signals from the second stimulation module. In the BTE unit, the additional antenna can be mounted in or on the ear hook.

In one embodiment of this aspect, the first and at least second stimulating signals can be provided to the same ear. In another embodiment, the first stimulating signal can be provided to one ear and the second stimulating signal to the other ear.

The first stimulation module can comprise an implantable component of a cochlear implant and so provides appropriate stimulation to the cochlea of a user. The implantable component of the cochlear implant can comprise a housing for the second transceiver or first receiver, a stimulator unit, at least one antenna, such as an antenna coil, and an electrode assembly. The electrode assembly can comprise an intracochlear electrode assembly that delivers stimulation to the auditory nerve of the implantee so producing a hearing sensation corresponding or representative of the original detected sound.

The intracochlear electrode assembly can comprise a carrier member having a leading end that is insertable into a cochlea of the implantee and a trailing end. The carrier can be formed of an elastomeric material, for example a silicone rubber material. The carrier member can have one or a plurality of electrodes mounted thereon. In one embodiment, the electrodes are mounted in a longitudinal array. Each of the electrodes can have at least one wire, for example two, extending from each electrode back towards the trailing end of the carrier member and then through a cable that extends back to the housing of the implantable component.

The carrier can have 22 electrodes. In another embodiment, the carrier can have 30 electrodes. Other numbers of electrodes can be utilised, including less than 20 electrodes, between 20 and 30 electrodes, and more 30 electrodes. The electrodes can be formed from a biocompatible electrically conducting material, such as platinum.

In another embodiment, the implantable component can have a second electrode assembly extending from the housing. The second electrode assembly may have one or more electrodes. This electrode assembly can be mounted within or external the cochlea of the implantee.

In one embodiment, the housing of the implantable component can be positioned subcutaneously and, if necessary, within a recess in the temporal bone of the implantee. The housing can be formed from a biocompatible material, for example, titanium. This housing can in turn be encapsulated in an electrically insulating surround. In a further embodiment, one or more hermetic and electrically insulated feedthroughs can be provided in the housing. The feedthroughs provide signal connection through the housing to the at least one antenna coil and/or the first and/or second electrode assembly. Power for the implantable component can be provided using the magnetic induction radio frequency link. In another embodiment, the implantable component can have an onboard rechargeable power source that is usable for example when an external component is not being used.

In another embodiment, the first stimulation module can comprise an actuator arrangement configured to provide direct stimulation to one or more hearing structures of the implantee representative of the original detected sound. The actuator can comprise at least one extracochlear actuator and be configured to provide excitation to fluid-filled inner-ear spaces of the implantee. In another embodiment, the actuator can comprise at least one intracochlear array of actuators. The actuator can comprise one or more mechanical actuation elements. The first stimulation module can comprise a direct acoustic cochlear actuator (DACS) unit.

In a still further embodiment, the first stimulation module can comprise a bone anchored hearing aid (or BAHA). The hearing aid can comprise a bone transmitting vibrator that is connected directly to the bone using a skin-penetrating and bone-anchored implant of a suitable biocompatible material, for example titanium.

In another embodiment of this aspect, the second stimulation module can comprise an acoustic/mechanical stimulation module that delivers mechanical stimulation in a range of audio frequencies. In one embodiment, the acoustic/mechanical stimulation module can comprise a completely-in-canal (CIC) unit that is shaped to fit into the ear canal of the implantee. The CIC unit can be positionable in the ear canal so as to touch the bony portion of the ear canal. In another embodiment, the acoustic/mechanical stimulation module can comprise an in-the-ear (ITE) unit that fits within the ear bowl. In one embodiment, the ITE unit can partially or wholly seal the ear bowl. In a still further embodiment, the acoustic/mechanical stimulation module can comprise an in-the-canal (ITC) unit. The ITC unit can be sized to not fit the ear bowl but instead be positionable just outside the ear canal. In yet another embodiment, the acoustic/mechanical stimulation module can comprise a micro-canal (MIC) unit. The MIC unit is typically slightly smaller in size than the ITC unit.

In addition to the third transceiver or second receiver, the second stimulation module can have an antenna that feeds signals to the second receiver or to and/or from the third transceiver, an on-board processor, a power source and an electro-acoustic transducer. The power source can comprise one or more batteries. The electro-acoustic transducer can comprise a speaker. If size permits, one or more controls can be provided on the second stimulation module.

The respective transceivers, transmitters or receivers and the antennae of the hearing prosthesis can operate together to provide wireless links between the components. Antennae can be formed so as to provide magnetic induction radio frequency links or electromagnetic radio frequency links. In another embodiment, the wireless link can be provided by a time domain multiple access (TDMA) system.

According to a second aspect, the present description is an auditory prosthesis comprising:
an implantable first stimulation module containing:
   a microphone for receiving and converting detected sounds into an output signal;
   a processor that converts and processes the output signal into a first stimulating signal representative of the detected sound; and
   a first transceiver or first transmitter that at least wirelessly transmits the digital signal; and
a second stimulation module having at least a second transceiver or first receiver that receives and processes the transmitted digital signal into a second stimulating signal representative of the detected sound.

In one embodiment, the first stimulation module is fully implantable and is configured to operate, at least for a period of time, not in conjunction with any external component. In one embodiment, the first stimulation module can have the features of the first stimulation module as defined with respect to the first aspect but modified as necessary so as to be able to operate in such a stand-alone manner. In addition to the microphone and processor, the implantable component can have an on-board power source.

When an external component is available and mounted appropriately, the implantable stimulation module can work in conjunction with that external component. For example, if the external component has a microphone and/or processor, the output of the external processor can be used instead of the on-board microphone and/or processor. When the external component comprises or has an on-board and suitable power source, the implantable component can be configured to preferentially draw power from the external component to power its operation and/or re-charge the on-board power source.

The first transceiver or first transmitter can transmit the digital signal via at least one antenna. In one embodiment, a single antenna can be provided on or with the implantable first stimulation module. The same antenna can be used to transmit to and receive signals from the second stimulation module and also be used as part of a transcutaenous link with an external component, when present. In another embodiment, two or more antennae can be provided on or with the first stimulation module. One of the two or more antennae can comprise a coil antenna and be used as part of a radio frequency magnetic induction link with a coil antenna of an external component, when used. Another one of the two or more antennae can provide a wireless link that is again transcutaneous to the second stimulation module.

In the second aspect, the second stimulation module can have one, some or all of the features of the second stimulation module as defined herein with respect to the first aspect. The second stimulation module can be operable to work in conjunction with the first stimulation module on the same ear of the implantee. In another embodiment, the respective stimulation modules can work on separate ears of the implantee.

According to a third aspect, the present description is an auditory prosthesis comprising:
a first component having:
   a microphone for receiving and converting detected sounds into an output signal;
   a processor that converts and processes the output signal into a digital signal; and
   a first transceiver or first transmitter that wirelessly transmits the digital signal;
an implantable first stimulation module having a second transceiver that:
   receives the transmitted digital signal from the first transceiver or first transmitter and processes this signal into a first stimulating signal representative of the detected sound; and
   re-transmits the transmitted digital signal; and
a second stimulation module having at least a third transceiver or first receiver that receives and processes the re-transmitted digital signal received from the first stimulation module into a second stimulating signal representative of the detected sound.

In the third aspect, the first component can comprise an external component. The external component can have one or more features of the external component as defined herein with respect to the other aspects. For example, the external component can comprise a BTE unit or ITE unit. The external component can have an on-board power source and/or be electrically connectable to a power source. In this aspect, the external component can have two or more antennae. One of the two or more antennae can comprise a coil antenna and be used as part of a radio frequency magnetic induction link with a coil antenna of the first stimulation module. This transcutaneous link can be used to power the first stimulation module and/or recharge a power source that is on-board the first stimulation module. Another one of the two or more antennae can provide a transcutaneous wireless link with a separate antenna on the implantable first stimulation module to allow transmission and reception of said digital signals.

Said separate antenna of the first stimulation module can be used to transcutaneously re-transmit the digital signal to the second stimulation module.

In the third aspect, the first stimulation module can have one, some or all of the features of the first stimulation module as defined herein with reference to other aspects. For example, the first stimulation module can comprise an implantable component of a cochlear implant, a direct acoustic cochlear actuator (DACS) unit, or a bone anchored hearing aid (or BAHA).

In the third aspect, the second stimulation module can have one, some or all of the features of the second stimulation module as defined herein with reference to other aspects. For example, the second stimulation module can comprise an ITE unit, a CIC unit, an ITC unit or a MIC unit.

In the third aspect, the second stimulation module can be operable to work in conjunction with the first stimulation module on the same ear of the implantee. In another embodiment, the respective stimulation modules can work on separate ears of the implantee.

It will be appreciated by persons skilled in the art that numerous variations and/or modifications may be made to the description as shown in the specific embodiments without departing from the scope of the invention as broadly described. The present embodiments are, therefore, to be considered in all respects as illustrative and not restrictive. The invention is set out in the appended claims :

## Claims

1. An auditory prosthesis comprising:
(I) a processor unit (400) comprising:
(a) a microphone (150, 440, 750) for receiving and converting detected sounds into an output signal;
(b) a processor that converts and processes the output signal into a first digital signal; and
(c) a first transceiver or first transmitter that wirelessly transmits the first digital signal;
(II) an implantable first stimulation module (100) adapted to output a first stimulating signal representative of the detected sounds, and
(III) at least one second stimulation module (200) adapted to output a second stimulating signal based on wirelessly received sound information according to the first digital signal,
wherein
at least one of the first and second stimulation modules (100, 200) comprises at least one of a transceiver and a receiver that receives and processes the first digital signal to achieve at least one of said first and second stimulating signal.

2. The auditory prosthesis of claim 1 wherein the implantable first stimulation module (100) comprises at least a second transceiver or first receiver that wirelessly receives the first digital signal and processes the first digital signal into said first stimulating signal representative of the detected sounds, and
wherein the at least one second stimulation module (200) comprises at least a third transceiver or second receiver that wirelessly receives and processes said first digital signal into a second stimulating signal representative of the detected sounds.

3. The auditory prosthesis of claim 1 wherein the implantable first stimulation module (100) includes the processor unit (400), and
wherein the at least one second stimulation module (200) comprises at least a third transceiver or second receiver that wirelessly receives and processes said first digital signal into a second stimulating signal representative of the detected sounds.

4. The auditory prosthesis of claim 1 wherein the implantable first stimulation module (100) comprises at least a second transceiver or first receiver that wirelessly receives the first digital signal and processes the first digital signal into said first stimulating signal and a second digital signal representative of the detected sounds and wherein the at least one second stimulation module (200) comprises at least a third transceiver or second receiver that wirelessly receives and processes said second digital signal into a second stimulating signal representative of the detected sounds.

5. The auditory prosthesis of any one of the preceding claims wherein the microphone (440, 750), processor and first transceiver or first transmitter are part of an external component having a housing, the external component is selected from group comprising a behind-the-ear (BTE) unit, an in-the-ear (ITE) unit, a pinna attached unit, or a body worn unit.

6. The auditory prosthesis of any one of the preceding claims wherein the first stimulation module comprises at least one of a cochlear implant, an actuator arrangement configured to provide direct stimulation to one or more hearing structures of an implantee, a direct acoustic cochlear actuator (DACS) unit, and a bone anchored hearing aid (BAHA).

7. The auditory prosthesis of claim 6 wherein the cochlear implant includes an intracochlear electrode assembly comprising a carrier member having a leading end that is insertable into a cochlea of the implantee and a trailing end, the carrier being formed of an elastomeric material and having a plurality of biocompatible electrodes mounted thereon.

8. The auditory prosthesis of claim 6 wherein the actuator arrangement comprises at least one extracochlear actuator that is arrangeable to provide at least one of mechanical excitation to fluid-filled inner-ear spaces, an electrical excitation of the auditory nerve at the outside of the cochlea.

9. The auditory prosthesis of claim 6 wherein the bone anchored hearing aid comprises a bone transmitting vibrator that is connected directly to the bone using a skin-penetrating and bone-anchored implant of a biocompatible material.

10. The auditory prosthesis of any one of the preceding claims wherein the second stimulation module comprises an acoustic/mechanical stimulation module that delivers mechanical stimulation in a range of audio frequencies,
wherein the acoustic/mechanical stimulation module is selected from the group comprising:
a completely-in-canal (CIC) unit that is shaped to fit into the ear canal of the implantee;
an in-the-ear (ITE) unit that fits within the ear bowl;
an in-the-canal (ITC) unit; and
a micro-canal (MIC) unit.

11. The auditory prosthesis of any one of the preceding claims wherein each of the respective transceivers, transmitters or receivers are provided with one or more antennae to provide wireless links between the components of the prosthesis, wherein at least one of the wireless links is provided preferably by a time domain multiple access (TDMA) system.

12. The auditory prosthesis of claim 11 wherein a wireless link from the external component is used to power at least one of the first and second stimulation module and/or recharge a power source that is on-board the first or second stimulation module.

13. The auditory prosthesis of any one of claims 11 or 12 wherein another one of the one or more antennae provides a transcutaneous wireless link with a separate antenna on the implantable first stimulation module to allow transmission and reception of said first and second digital signals.

14. The auditory prosthesis of claim 13 wherein said separate antenna of the first stimulation module is used to transcutaneously re-transmit the first and second digital signal to the second stimulation module.

15. The auditory prosthesis of any one of the preceding claims wherein the second stimulation module is operable to work in conjunction with the first stimulation module on the same ear or on separate ears of the implantee.

## Patentansprüche

1. Hörprothese, umfassend:
(I) eine Prozessoreinheit (400), umfassend:
(a) ein Mikrofon (150, 440, 750) zum Empfangen und Umwandeln erfasster Töne in ein Ausgangssignal;
(b) einen Prozessor, der das Ausgangssignal umwandelt und zu einem ersten Digitalsignal verarbeitet; und
(c) einen ersten Sendeempfänger oder ersten Sender, der das erste Digitalsignal drahtlos sendet;
(II) ein implantierbares erstes Stimulationsmodul (100), das dazu eingerichtet ist, ein erstes Stimulationssignal auszugeben, das für die erfassten Töne repräsentativ ist; und
(III) wenigstens ein zweites Stimulationsmodul (200), das dazu eingerichtet ist, ein zweites Stimulationssignal auf der Basis drahtlos empfangener Toninformationen gemäß dem ersten Digitalsignal auszugeben,
wobei
das erste und/oder das zweite Stimulationsmodul (100, 200) einen Sendempfänger und/oder einen Empfänger umfassen/umfasst, der das erste Digitalsignal empfängt und verarbeitet, um das erste und/oder das zweite Stimulationssignal zu erzielen.

2. Hörprothese nach Anspruch 1, beider das implantierbare erste Stimulationsmodul (100) wenigstens einen zweiten Sendeempfänger oder einen ersten Empfänger umfasst, der das erste Digitalsignal drahtlos empfängt und das erste Digitalsignal zu dem ersten Stimulationssignal verarbeitet, das für die erfassten Töne repräsentativ ist; und
das wenigstens eine zweite Stimulationsmodul (200) wenigstens einen dritten Sendeempfänger oder einen zweiten Empfänger umfasst, der das erste Digitalsignal empfängt und zu einem zweiten Stimulationssignal verarbeitet, das für die erfassten Töne repräsentativ ist.

3. Hörprothese nach Anspruch 1, bei der das implantierbare erste Stimulationsmodul (100) die Prozessoreinheit (400) umfasst, und
das wenigstens eine zweite Stimulationsmodul (200) wenigstens einen dritten Sendeempfänger oder zweiten Empfänger umfasst, der das erste Digitalsignal drahtlos empfängt und zu einem zweiten Stimulationssignal verarbeitet, das für die erfassten Töne repräsentativ ist.

4. Hörprothese nach Anspruch 1, bei der das implantierbare erste Stimulationsmodul (100) wenigstens einen zweiten Sendempfänger oder ersten Empfänger umfasst, der das erste Digitalsignal drahtlos empfängt und das erste Digitalsignal zu dem ersten Stimulationssignal und ein zweites Digitalsignal verarbeitet, das für die erfassten Töne repräsentativ ist; und
das wenigstens eine zweite Stimulationsmodul (200) wenigstens einen dritten Sendeempfänger oder zweiten Empfänger umfasst, der das zweite Digitalsignal drahtlos empfängt und zu einem zweiten Stimulationssignal verarbeitet, das für die erfassten Töne repräsentativ ist.

5. Hörprothese nach einem der vorhergehenden Ansprüche, bei der das Mikrofon (440, 750), der Prozessor und der erste Sendeempfänger oder der erste Sender Teil eines externen Bauteils sind, das ein Gehäuse hat, wobei das externe Bauteil aus einer Gruppe gewählt ist, die eine Hinter-Ohr- (BTE-) Einheit, eine In-Ohr- (ITE-) Einheit, eine an der Ohrmuschel angebrachte Einheit oder eine am Körper getragene Einheit umfasst.

6. Hörprothese nach einem der vorangehenden Ansprüche, bei der das erste Stimulationsmodul ein Cochlearimplantat und/oder eine Stellgliedanordnung, die dazu eingerichtet ist, eine direkte Stimulation für eine oder mehrere Hörstrukturen eines Implantatsträgers bereitzustellen, und/oder eine direkte akustische Cochlearstellglied-(DACS-) Einheit und/oder eine knochenverankerte Hörhilfe (BAHA) umfasst.

7. Hörprothese nach Anspruch 6, bei der das Cochlearimplantat eine intracochleare Elektrodenanordnung umfasst, die ein Trägerelement umfasst, das ein vorderes Ende, das in eine Cochlea des Implantatsträgers eingefügt werden kann, und ein hinteres Ende hat, wobei der Träger aus einem Elastomermaterial besteht und auf ihm eine Vielzahl von biokompatiblen Elektroden angebracht ist.

8. Hörprothese nach Anspruch 6, bei der die Stellgliedanordnung wenigstens ein extracochleares Stellglied umfasst, das so eingerichtet sein kann, dass es eine mechanische Anregung an fluidgefüllten Innenohrräumen und/oder eine elektrische Anregung an dem Hörnerv auf der Außenseite der Cochlea hervorrufen kann.

9. Hörprothese nach Anspruch 6, bei der die knochenverankerte Hörhilfe einen Knochenübertragungsvibrator umfasst, der direkt mit dem Knochen mit Hilfe eines hautdurchdringenden und knochenverankerten Implantates eines biokompatiblen Materials verbunden ist.

10. Hörprothese nach einem der vorangehenden Ansprüche, bei der das zweite Stimulationsmodul ein akustisches/mechanisches Stimulationsmodul umfasst, das eine mechanische Stimulation in einem Bereich von Audiofrequenzen liefert,
wobei das akustische/mechanische Stimulationsmodul aus der Gruppe gewählt ist, die umfasst:
eine Komplett-in-Kanal- (CIC-) Einheit, die derart ausgeformt ist, dass sie in den Ohrkanal des Implantatsträgers passt;
eine In-Ohr- (ITE-) Einheit die in die Ohrmuschel passt;
eine In-Kanal- (ITC-) Einheit; und
eine Mikro-Kanal- (MIC-) Einheit.

11. Hörprothese nach einem der vorangehenden Ansprüche, bei der jeder der entsprechenden Sendeempfänger, Sender oder Empfänger mit wenigstens einer Antenne ausgestattet ist, um drahtlose Verbindungen zwischen den Bauteilen der Prothese herzustellen, wobei wenigstens eine der drahtlosen Verbindungen vorzugsweise durch ein Zeitmultiplexier- (TDMA-) System bereitgestellt ist.

12. Hörprothese nach Anspruch 11, bei der eine drahtlose Verbindung von dem externen Bauteil verwendet wird, um das erste und/oder das zweite Stimulationsmodul mit Strom zu versorgen und/oder eine Stromquelle wiederaufzuladen, die sich in dem ersten oder dem zweiten Stimulationsmodul befindet.

13. Hörprothese nach Anspruch 11 oder 12, bei der eine weitere der einen oder der mehreren Antennen eine transkutane drahtlose Verbindung mit einer separaten Antenne auf dem implantierbaren ersten Stimulationsmodul bereitstellt, um eine Sendung und einen Empfang der ersten und zweiten Digitalsignale zu gestatten.

14. Hörprothese nach Anspruch 13, bei der die separate Antenne des ersten Stimulationsmoduls verwendet wird, um transkutan das erste und das zweite Digitalsignal zu dem zweiten Stimulationsmodul erneut zu senden.

15. Hörprothese nach einem der vorangehenden Ansprüche, bei der das zweite Stimulationsmodul derart betätigt werden kann, dass es in Verbindung mit dem ersten Stimulationsmodul an demselben Ohr oder an getrennten Ohren des Implantatsträgers arbeitet.

## Revendications

1. Prothèse auditive comprenant .
(I) une unité de processeur (400) comprenant :
(a) un microphone (150, 440, 750) destiné à recevoir et à convertir des sons détectés en un signal de sortie,
(b) un processeur qui convertit et traite le signal de sortie en un premier signal numérique, et
(c) un premier émetteur récepteur ou un premier émetteur qui transmet par communication sans fil le premier signal numérique,
(II) un premier module de stimulation implantable (100) conçu pour délivrer en sortie un premier signal de stimulation représentatif des sons détectés, et
(III) au moins un second module de stimulation (201) conçu pour délivrer en sortie un second signal de stimulation fondé sur des informations de sons reçues sans fil en fonction du premier signal numérique,
dans laquelle
au moins l'un des premier et second modules de stimulation (100, 200) comprend au moins l'un d'un émetteur récepteur et d'un récepteur qui reçoit et traite le premier signal numérique afin d'obtenir au moins l'un desdits premier et second signaux de stimulation.

2. Prothèse auditive selon la revendication 1, dans laquelle le premier module de stimulation implantable (100) comprend au moins un deuxième émetteur récepteur ou un premier récepteur qui reçoit sans fil le premier signal numérique et traite le premier signal numérique en dit premier signal de stimulation représentatif des sons détectés, et
dans laquelle au moins un second module de stimulation (200) comprend au moins un troisième émetteur récepteur ou un deuxième récepteur qui reçoit sans fil et traite le premier signal numérique en un second signal de stimulation représentatif des sons détectés.

3. Prothèse auditive selon la revendication 1, dans laquelle le premier module de stimulation implantable (100) inclut l'unité de processeur (400), et
dans lequel le ou les seconds modules de stimulation (200) comprennent au moins un troisième émetteur récepteur ou un deuxième récepteur qui reçoit sans fil et traite ledit premier signal numérique en un second signal de stimulation représentatif des sons détectés.

4. Prothèse auditive selon la revendication 1, dans laquelle le premier module de stimulation implantable (100) comprend au moins un deuxième émetteur récepteur ou un premier récepteur qui reçoit sans fil le premier signal numérique et traite le premier signal numérique en dit premier signal de stimulation ainsi qu'un second signal numérique représentatif des sons détectés, et
dans lequel le ou les seconds modules de stimulation (200) comprennent au moins un troisième émetteur récepteur ou un second récepteur qui reçoit sans fil et traite ledit second signal numérique en un second signal de stimulation représentatif des sons détectés.

5. Prothèse auditive selon l'une quelconque des revendications précédentes, dans laquelle le microphone (440, 750), le processeur et le premier émetteur récepteur ou le premier émetteur font partie d'un composant externe possédant une enveloppe, le composant externe étant sélectionné à partir du groupe comprenant une unité contour d'oreille (BTE), une unité interne à l'oreille (ITE), une unité fixée au pavillon ou une unité portée sur le corps.

6. Prothèse auditive selon l'une quelconque des revendications précédentes, dans laquelle le premier module de stimulation comprend au moins l'un d'un implant cochléaire, d'un agencement formant actionneur configuré pour fournir une stimulation directe à une ou plusieurs structures d'écoute d'une personne porteuse d'implant, d'une unité d'actionneur cochléaire acoustique direct (DACS) et d'une assistance à l'écoute ancrée dans l'os (BAHA).

7. Prothèse auditive selon la revendication 6, dans laquelle l'implant cochléaire inclut un ensemble formant électrode intra cochléaire comprenant un élément de support possédant une extrémité avant qui peut être insérée dans une cochlée de la personne porteuse d'implant et une extrémité arrière, le support étant formé d'un matériau élastomère et comportant une pluralité d'électrodes biocompatibles montées sur lui.

8. Prothèse auditive selon la revendication 6, dans lequel l'agencement formant actionneur comprend au moins un actionneur extra cochléaire qui peut être agencé pour fournir au moins l'une d'une excitation mécanique vers les espaces de l'oreille interne remplis de fluide, et d'une excitation électrique du nerf auditif à l'extérieur de la cochlée.

9. Prothèse auditive selon la revendication 6, dans laquelle l'assistance à l'écoute ancrée dans l'os comprend un élément vibrant de transmission à l'os qui est directement relié à l'os en utilisant un implant pénétrant dans la peau et ancré dans l'os constitué d'un matériau biocompatible.

10. Prothèse auditive selon l'une quelconque des revendications précédentes, dans laquelle le second module de stimulation comprend un module de stimulation acoustique et mécanique qui délivre une stimulation mécanique dans la plage des fréquences audio,
dans laquelle le module de stimulation acoustique et mécanique est sélectionné à partir du groupe comprenant :
une unité intégralement dans le canal (CIC) qui prend une forme lui permettant de s'ajuster dans le canal auditif de la personne porteuse d'implant,
une unité intra oreille (ITE) qui s'ajuste à l'intérieur de la cavité de l'oreille,
une unité intra canal (ITC), et
une unité micro canal (MIC)

11. Prothèse auditive selon l'une quelconque des revendications précédentes, dans laquelle chacun des émetteurs récepteurs, des émetteurs ou des récepteurs respectifs sont munis d'une ou de plusieurs antennes permettant de procurer des liaisons sans fil entre les composants de la prothèse,
dans laquelle au moins l'une des liaisons sans fil est fournie de préférence grâce à un système à accès multiple dans le domaine du temps (TDMA).

12. Prothèse auditive selon la revendication 11, dans laquelle une liaison sans fil provenant du composant externe est utilisée pour alimenter au moins l'un du premier et du second module de stimulation et/ou pour recharger une source d'énergie qui est embarquée sur le premier ou le second module de stimulation.

13. Prothèse auditive selon l'une quelconque des revendications 11 ou 12, dans laquelle une autre de la ou des antennes fournit une liaison sans fil transcutanée avec une antenne séparée sur le premier module de stimulation implantable afin de permettre l'émission et la réception desdits premier et second signaux numériques.

14. Prothèse auditive selon la revendication 13, dans laquelle ladite antenne séparée du premier module de stimulation est utilisée pour retransmettre par voie transcutanée les premier et second signaux numériques au second module de stimulation.

15. Prothèse auditive selon l'une quelconque des revendications précédentes, dans laquelle le second module de stimulation peut être mis en oeuvre pour fonctionner conjointement avec le premier module de stimulation sur la même oreille ou sur des oreilles différentes de la personne porteuse d'implant.
